# EUROPEAN PATENT SPECIFICATION

(11) **EP 2 185 229 B1**
(45) Date of publication and mention of the grant of the patent: **05.12.2012**
(21) Application number: 08827042.6
(22) Date of filing: 01.08.2008
(51) Int. Cl.: A61M 16/04

(54) **ENDOTRACHEAL TUBE**
ENDOTRACHEALTUBUS
TUBE ENDOTRACHÉAL

(30) Priority: 06.08.2007 IT BO20070565
(43) Date of publication of application: 19.05.2010
(73) Proprietor: Medical Service S.r.l., 84131 Salerno (IT)
(72) Inventor: AITA, Giuseppe, I-84123 Salerno (IT)
(74) Representative: Barberi, Vittorio
(86) International application number: PCT/IT2008/000530
(87) International publication number: WO 2009/019734

(56) References cited:
- EP-A- 0 131 659
- WO-A-95/32017
- US-A- 4 444 185
- US-A- 4 722 335
- US-A- 4 979 505
- US-A- 5 285 778
- US-A1- 2002 108 610

## Description

The present invention relates to an endotracheal tube.

In particular, the tube of the invention is usable for the endotracheal intubation prior to the execution of percutaneous tracheotomy.

It is known that the tracheostomy is a surgical technique used for creating a link between the trachea and the outside, in case of patients with primary or secondary respiratory diseases.

The main advantages of this technique relate to a reduction in respiratory dead space, to a better adaptation to a possible assisted ventilation, to facilitate the operations of "nursing" and "weaning" stages of the respirator. All this affects favourably faster healing.

Among the known techniques for the tracheostomy there are, for example: the classic surgical technique, which provides surgical dissection of all plans of the neck and tracheotomy with subsequent placement of a special cannula; the retrograde percutaneous technique according to Fantoni, which is realized intubating the patient with a tracheoscope which ensures also the ventilation; the dilating percutaneous technique according to Ciaglia, which consists in introducing a needle in the trachea after the manual search of the cutaneous repere point and the subsequent placement into the trachea of probes having size gradually growing before the introduction of the tracheotomic cannula.

The techniques mentioned above have various drawbacks.

Among these, in particular, there is the search for the repere, i.e. the point in which the transcutaneous puncture has to be executed: this operation is executed in an entirely manual way and without any guide: for this reason it is possible that during the introduction of the needle, the same needle can be introduced in a blood vessel not detectable. Another risk relevant to the state of the art is the possible accidental puncture of the rear wall of the trachea.

The present invention is relevant to the tracheostomy percutaneous methods involving the use of a particular tube for tracheal intubation, which can be made, for example, using PVC, silicone PVC or other material, and having size (length and diameter) similar to the usual tubes for tracheal intubation and provided with a "standard" connector for connecting to artificial ventilation circuits. This type of endotracheal tube is provided with: a distal end which is shaped as a little plate or "palette", open at the front; an inflatable element or "cuff", connected to an inflation system; means of illumination.

The lighting means of this type of device may include a high brightness micro-lamp, which can be powered by low-voltage direct current, with external battery. Alternatively, can be used an optical fibres system with external light source.

By this device, the percutaneous tracheotomy is performed with relatively high safety and with considerable ease. This embodiment further improves the solution described above thanks to the particular positioning of the lighting means, which are substantially in contact with the wall of the trachea, without any interposed obstacle.

Furthermore, according to an embodiment, it is possible to activate the lighting means of the tube without providing the tube with electric power cables, further simplifying the structure of the endotracheal tube.

Advantageously, the intubation realized by the tube of the invention allows, in addition to an optimal ventilation during all phases of the operation, to identify without errors the point for inserting a needle into trachea under the guide of a light beam. By inserting the guide needle few millimeters below the trans-lighting beam, which is visible on the skin, it is possible to proceed in surgical technique using all conventional dilating methods. In addition, the insertion of the guide needle and the dilating devices is total safety as the back wall of the trachea is protected by the palette previously described. This result has been achieved according to the invention thanks to the idea of actuating an endotracheal tube having the features described in the independent claim. Other features relate to the dependant claims.

Among the advantages of this invention can be listed those described below:
- precise location of the point of surgical attack even in condition of deviation of the trachea, thanks to the trans-illumination emitted in close contact with the (front) wall of the trachea;
- absence of supply lines along the development of the tube, thanks to the radio-frequency power supply provided with transcutaneous transmitter;
- protection of the rear wall of the trachea in all phases of the operation, from the insertion of the needle to the whole phase of expansion, thanks to the distal end shaped as an open palette;
- smooth insertion of dilating devices without the need to change the position of the tube, reducing in this way the possibility of accidental disengagement of the intubation;
- guarantee of ventilation at every stage, with a tube of adequate size.

These and other advantages and characteristics of the invention will be best understood by anyone skilled in the art from a reading of the following description in conjunction with the attached drawings given as a practical exemplification of the invention, but not to be considered in a limitative sense, wherein:
- Fig.1 is a schematic view in which is shown an example of use of this invention;
- Fig.2 is a schematic side view in which the endotracheal tube is partially represented, divided into several portions;
- Figs.3 and 4 are cross sections, in which are schematically represented two possible embodiments of the invention.

With reference to the examples of attached drawings, an endotracheal tube 1 according to the invention essentially consists of a tubular hollow body 10 which can be made, for example, using PVC, silicone PVC or other suitable materials.

In the example of Fig,2, the tube 1 are provided, in its proximal end, with a connector 5 of known type which allows the connection to apparatuses for assisted ventilation.

The distal end, to be inserted in trachea, is provided with means 3 suitable to emit a light radiation for determining the transcutaneous illumination (in this description said means are called also lighting means) and variable volume means 2, which are integral with the body of the tube 1 and are suitable to vary the total diameter of the same tube, i.e. its encumbrance in the lumen of the trachea.

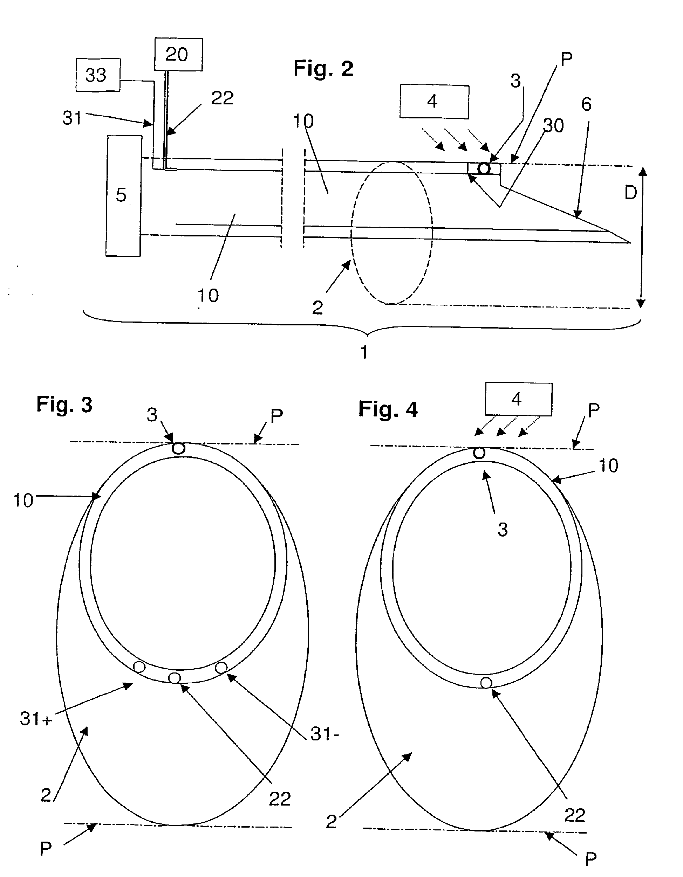

In Figs.2-4 the letter P represents the walls of the trachea and D the value of the diameter of the relevant lumen. These representations are schematic.

The variable volume means 2 are shaped and/or positioned so that their expansion maintain said lighting means 3 lighting substantially near the tracheal wall P, i.e. in a position in which the means 3 are disposed substantially in contact with a wall P of the same trachea.

In practice, said variable volume means 2 include an inflatable element, also called balloon or cuff, which, once inflated, expands only on one side in respect to the longitudinal axis of the tube 10, with the lighting means 3 disposed on the opposite side. The balloon 2 is connected to appropriate means 20 for its inflation through a conduit 22 (visible also in Figs.3 and 4). Although in the examples of drawings the numeral reference 2 indicates a balloon, the expansion within the lumen D of the trachea is achievable even with a device such as variable diameter "stent", which in this case, according to the invention, will expand substantially exclusively to one side of the endotracheal tube, that is the side opposite to the lighting means 3.

As shown in Figs. 2 and 4, the means suitable to emit light radiation 3 include a circuit 30 which can be activated trough a radio frequency signal, by a corresponding transmitter 4 which sends a transcutaneous transmission signal. In other words, the circuit 30 is able to transform into a current the signal received by transcutaneous way for supplying the lighting means 3.

The lighting means, advantageously, may be of LED type. By using radio frequency transmission the cables for the power supply of lighting means are eliminated.

In Fig.2 the block 33 represents a possible external source such as a battery, and 31 represents the relevant connection to the lighting means 3.

Similarly, in the example of Fig.3, references 31+ and 31-represent the power supply cables used without the radio frequency device.

The tube 1, as many tubes of known type, has its distal end 6 which is shaped as an open palette.

During installation of the tube 1, a needle is initially inserted and, then, a wire is inserted through this needle. The subsequent phases of expansion will be carried out smoothly, since the palette 6 is open upwards and then there is not any obstacle in the insertion of the dilating devices. The proper lubrication of the palette will not hinder in any way the initial insertion of various expansion probes which are steadily increasing in size and the subsequent insertion of the tracheostomic cannula.

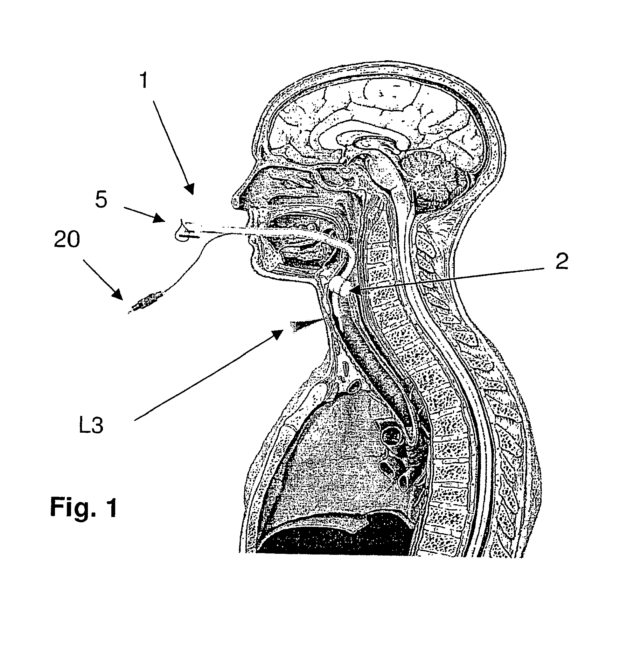

In Fig.1, L3 represents the light beam emitted by the lighting means 3; this beam allows the smooth introduction of the needle, as previously described.

The command and control organs of the above elements illustrated in the enclosed drawings are well-known to the technicians of this field and have not been described in more detail for simplicity's sake. Moreover, the execution details may equally vary as regards shape, size, disposition of elements, kind of material used, within the limits of the solution idea that has been adopted and within the limits of the present invention.

## Claims

1. Endotracheal tube (1), usable in dilating percutaneous tracheostomy operations and comprising a tubular body (10) whose distal end, to be inserted in trachea, is provided with means (3) suitable to emit a light radiation for determining a transcutaneous illumination and with variable volume means (2), integral to said tube (1) and suitable to vary the total diameter of the tube, that is its encumbrance in the lumen of the trachea; tube (1) **characterized in that** said variable volume means (2) are shaped and/or positioned so that their expansion maintains said means (3) suitable to emit a radiation light substantially near the tracheal wall (P), that is in a position in which the means (3) are disposed substantially in contact with a wall (P) of the same trachea and **in that** said variable volume means (2) include an inflatable element which, once inflated, expands only on one side in respect to the longitudinal axis of the tube (10), said means suitable to emit a light radiation (3) being disposed on the opposite side.

2. Tube according to claim 1, **characterized in that** said means suitable to emit light radiation (3) include a circuit (30) activated trough radio frequency, by a corresponding transmitter (4) for the transcutaneous transmission of the relevant signal.

3. Tube according to claim 1 or 2, **characterized in that** said means for emitting light radiation (3) include a circuit (30) suitable to transform into a current a signal received by transcutaneous way in order to supply said means (3) suitable to emit a light radiation.

4. Tube according to one of the previous claims **characterized in that** said lighting means (3) are of LED type.

5. Tube according to one of the previous claims **characterised in that** it has its distal shapes as an open palette (6).

## Patentansprüche

1. Endotrachealtubus (1), der bei der perkutanen Dilatationstracheotomie angewandt werden kann und einen röhrenförmige Körper (10) besitzt, dessen distales Endstück, das zur Einführung in die Luftröhre bestimmt ist, über Mittel (3), die eine hellleuchtende Strahlung ausüben, um eine transkutane Beleuchtung zu erzielen, und über Mittel von veränderlichem Volumen (2) verfügt, die eins mit dem besagten Tubus (1) und geeignet sind, den Gesamtumfang desselben Tubus oder den Platzbedarf im Luftröhrenlumen zu ändern; Tubus (1), **dadurch gekennzeichnet, dass** die besagten Mittel mit veränderbaren Volumen (2) so gestaltet und/oder positioniert sind, dass sie die besagten Mittel (3), die geeignet sind, eine hell leuchtende Strahlung im Wesentlichen auszuüben, in der Nähe der Trachealwand (P), oder in einer Position halten, in der die Mittel (3) sich im Wesentlichen in Kontakt mit der Trachealwand (p) befinden, und von der Tatsache, dass die besagten Mittel mit veränderbaren Volumen (2) ein aufblasbares Element umfasst, das sich nach dem Aufblasen gegenüber der Längsachse des Tubus (10) nur auf einer Seite ausdehnt, da die besagten Mittel, die dazu geeignet sind, eine hell leuchtende Strahlung (3) auszustoßen, sich auf der entgegengesetzten Seite befinden.

2. Tubus, gemäß Anspruch 1, **dadurch gekennzeichnet, dass** die besagten Mittel, die imstande sind, eine hell leuchtende Strahlung (3) auszustoßen, eine Schaltung (30) umfassen, die durch elektromagnetische Wellen aktiviert werden kann, da ein entsprechender Sender (4) für eine transkutane Übertragung des entsprechenden Signals vorgesehen ist.

3. Tubus gemäß Anspruch 1 oder 2, **dadurch gekennzeichnet, dass** die besagten Mittel, die imstande sind, eine hell leuchtende Strahlung (3) auszustoßen, eine Schaltung (30) umfassen, die imstande ist, ein transkutan empfangenes Signal in Strom umzuwandeln, so dass die besagten Mittel (3), die eine hell leuchtende Strahlung ausstoßen, gespeist werden.

4. Tubus gemäß einer der vorherigen Ansprüche, **dadurch gekennzeichnet, dass** die Leuchtmittel (3) LED-Leuchten sind.

5. Tubus gemäß einer der vorhergehenden Ansprüche, **dadurch gekennzeichnet, dass** das eigene distale Endstück die Form einer offenen Schaufel besitzt (6).

## Revendications

1. Tube endo-trachéal (1), utilisables dans les opérations de trachéotomie percutanée dilatante et comprenant un corps tubulaire (10) dont l'extrémité distale, destinée à être insérée dans la trachée, est pourvu de moyens (3) aptes à émettre un rayonnement de lumière pour déterminer une illumination transcutanée et avec des moyens à volume variable (2), qui font partie intégrante dudit tube (1) et adaptés pour faire varier le diamètre total du tube, c'est-à-dire leur encombrement dans la lumière de la trachée; tube (1) **caractérisé en ce que** lesdits moyens à volume variable (2) sont de forme et / ou positionné de telle sorte que leur extension maintient lesdits moyens (3) aptes à émettre un rayonnement lumineux substantiellement à proximité de la paroi de la trachée (P), c'est-à-dire dans une position dans laquelle les moyens (3) sont disposés substantiellement en contact avec un paroi (P) de la trachée même et **en ce que** lesdits moyens à volume variable (2) comprennent un élément gonflable qui, une fois gonflé, s'étend seulement sur un côté par rapport à l'axe longitudinal du tube (10), lesdits moyen aptes à émettre un rayonnement de lumière (3) étant disposé sur le côté opposé.

2. Tube selon la revendication 1, **caractérisé en ce que** lesdits moyens aptes à émettre un rayonnement de lumière (3) comprennent un circuit (30) activé par radiofréquence, par un correspondant émetteur (4) pour la transmission transcutanée du signal pertinent.

3. Tube selon la revendication 1 ou 2, **caractérisé en ce que** lesdits moyens de rayonnement émettant de la lumière (3) comprennent un circuit (30) apte à se transformer en courant un signal reçu par voie transcutanée afin de alimenter lesdites moyens (3) aptes à émettre un rayonnement lumineux.

4. Tube selon l'une quelconque des revendications précédentes, **caractérisé en ce que** lesdits moyens d'éclairage (3) sont de type LED.

5. Tube selon l'une quelconque des revendications précédentes, caractérisé en ce l'extrémité distale du tube a la forme d'une palette ouverte (6).
